# EUROPÄISCHE PATENTSCHRIFT

(11) **EP 2 707 047 B1**
(45) Veröffentlichungstag und Bekanntmachung des Hinweises auf die Patenterteilung: **12.08.2015**
(21) Anmeldenummer: 12715996.0
(22) Anmeldetag: 07.05.2012
(51) Int. Cl.: A61M 1/00

(54) **VORRICHTUNG ZUR BEREITSTELLUNG VON UNTERDRUCK ZUR MEDIZINISCHEN UNTERDRUCKBEHANDLUNG VON WUNDEN**
DEVICE FOR PROVIDING VACUUM FOR THE MEDICAL VACUUM TREATMENT OF WOUNDS
DISPOSITIF DE PRODUCTION D'UNE PRESSION NÉGATIVE POUR LE TRAITEMENT MÉDICAL DES PLAIES PAR PRESSION NÉGATIVE

(30) Priorität: 13.05.2011 DE 102011075842
(43) Veröffentlichungstag der Anmeldung: 19.03.2014
(73) Patentinhaber: Paul Hartmann AG, 89522 Heidenheim (DE)
(72) Erfinder: ECKSTEIN, Axel, 89522 Heidenheim (DE); HOFSTETTER, Juergen, 89522 Heidenheim (DE)
(74) Vertreter: DREISS Patentanwälte PartG mbB
(86) Internationale Anmeldenummer: PCT/EP2012/057251
(87) Internationale Veröffentlichungsnummer: WO 2012/156174

(56) Entgegenhaltungen:
- WO-A1-96/05873
- WO-A2-2009/021047
- GB-A- 2 342 584
- US-A1- 2008 200 857
- US-A1- 2009 240 218
- US-A1- 2011 106 058

## Beschreibung

Die Erfindung betrifft eine am Körper eines Benutzers tragbare Vorrichtung zur Bereitstellung von Unterdruck zur medizinischen Unterdruckbehandlung von Wunden am menschlichen oder tierischen Körper, umfassend eine unterdruckerzeugende Saugpumpe in einem ersten Gehäuseteil der Vorrichtung, einen Behälter zur Aufnahme von Körperflüssigkeiten, insbesondere von aus einer Wunde abgesaugten Wundsekreten, wobei der Behälter lösbar an dem ersten Gehäuseteil der Vorrichtung befestigbar ist und im befestigten Zustand von der Saugpumpe mit Unterdruck beaufschlagbar ist, und wobei am Behälter ein Anschluss für eine zum Körper führende Saugleitung vorgesehen ist, so dass eine Unterdruckkommunikation zwischen der Saugpumpe, dem Behälter und der zum Körper führenden Saugleitung herstellbar ist, eine programmierbare elektronische Steuereinrichtung, welche mindestens unter Berücksichtigung von vorgegebenen und/oder vorgebbaren Parametern und von durch den Drucksensor gemessenen Druckwerten die Saugpumpe ansteuern kann. Wenn vorstehend von einer tragbaren Vorrichtung die Rede ist, so bedeutet dies, dass der Patient die Vorrichtung mitführen kann, so dass er mobil ist und dennoch seine Wunde dauerhaft, d.h. ohne Unterbrechung, therapiert werden kann. Die tragbare Vorrichtung kann dabei über an sich beliebige Befestigungsmittel, insbesondere und vorzugsweise in Form eines flexiblen Gürtels oder eines Schultertragriemens, am Körper des Patienten gehalten und mitgeführt werden. Eine tragbare Vorrichtung der hier in Rede stehenden Art kann aber natürlich auch im stationären Betrieb, also losgelöst vom Körper des Patienten, eingesetzt werden; sie kann solchenfalls beispielsweise an einem Pflegebett befestigt oder neben dem Pflegebett abgestellt werden.

Vorrichtungen zur Unterdruckwundbehandlung sind bereits mehrfach beschrieben worden, insbesondere durch US 2004/0073151 A1, WO 2009/047524 A2, WO 2007/030599 A2 oder EP 1 905 465 A1, EP 777 504 B und durch DE 10 2009 038 130 A und DE 10 2009 038 131 A der Anmelderin.

WO 96/05873 offenbart eine Vorrichtung der eingangs genannten Art, bei der der Behälter in einen horizontalen Aufnahmeschacht einschiebbar ist. Beim Einschalten des Unterdruckbetriebs steuert die elektronische Steuereinrichtung die Saugpumpe nur an, wenn sie ein Signal von einem durch Einschieben des Behälters mechanisch betätigten Sensorschalter erhält, der somit die korrekte Behältermontage detektiert.

Bei Vorrichtungen zur Unterdruckbehandlung von Wunden kommuniziert eine Saugpumpe über eine Saugleitung mit der Wunde oder der Wundumgebung, wobei ein Wundverband mit einem luftundurchlässigen Abdeckmaterial zum luftdichten Verschließen der Wunde und der Wundumgebung vorgesehen ist, so dass ein Unterdruck im Wundraum herstellbar und Flüssigkeiten aus dem Wundraum in den genannten Behälter absaugbar sind.

Der Begriff eines Unterdrucks bezeichnet im Zusammenhang mit der vorliegenden Erfindung einen gegenüber dem Umgebungsluftdruck (atmosphärischer Luftdruck) erniedrigten Luftdruck insbesondere innerhalb eines Wundverbands. Das Abdeckmaterial eines Wundverbands zum luftdichten Verschließen eines Wundraums muss daher so ausgebildet sein, dass es der sich einstellenden Druckdifferenz standzuhalten vermag, damit der Unterdruck im Wundraum überhaupt angelegt und aufrechterhalten werden kann. Eine gewisse Nachgiebigkeit des Wundverbands und Abdeckmaterials ist jedoch typischerweise gegeben. Im Bereich der Unterdrucktherapie in der Wundbehandlung wird der Unterdruck quantitativ als Druckdifferenz zwischen dem Umgebungsluftdruck und dem unterhalb des Abdeckmaterials angelegten Luftdruck angegeben. Typischerweise beträgt diese Druckdifferenz im Bereich der Unterdrucktherapie höchstens 250 mmHg (mm Quecksilbersäule) (1 mm Hg = 1 Torr. = 133,322 Pa). Dieser Unterdruckbereich bis höchstens 250 mmHg hat sich als für die Wundheilung geeignet erwiesen. Ein bevorzugter Unterdruckbereich liegt zwischen 10 und 150 mmHg.

Der unter Verwendung der Vorrichtung an die Wunde angelegte Unterdruck kann bei einer typischen Unterdruckbehandlung entweder zeitlich im Wesentlichen konstant gehalten werden, oder er kann zeitlich verändert, insbesondere zyklisch verändert werden, was über eine entsprechend ausgebildete und programmierte Steuervorrichtung bei der Unterdruck erzeugenden Einrichtung, insbesondere in Abhängigkeit weiterer Parameter, realisiert werden kann.

Zum Anlegen von Unterdruck und vorzugsweise auch zum Absaugen von Körperflüssigkeiten ist eine vorzugsweise flexible Saugleitung, beispielsweise in Form eines Drainageschlauchs, vorgesehen, der einenends über einen sogenannten Port im Bereich des Wundabdeckmaterials mit der Wundumgebung oder dem Wundraum und anderenends mit dem eingangs erwähnten Behälter zur Aufnahme von Körperflüssigkeiten bzw. mit der Unterdruck erzeugenden Einrichtung kommuniziert.

Neben der Unterdruckwundbehandlung sind auch andere Anwendungen der hier in Rede stehenden Vorrichtung zur Bereitstellung von Unterdruck für medizinische Anwendungen denkbar, insbesondere die Absaugung an sich beliebiger Körperflüssigkeiten, im Bereich der medizinischen Inkontinentenversorgung, der Versorgung von Stoma-Patienten oder im Bereich der Absaugung von Wundsekreten, gegebenenfalls unter Verwendung von Spülflüssigkeiten, auch ohne Anlegen eines Unterdrucks über wesentliche Zeiträume.

Der vorliegenden Erfindung liegt, ausgehend von einer gattungsgemäßen tragbaren Vorrichtung zur Bereitstellung von Unterdruck für medizinische Anwendungen die Aufgabe zugrunde, die Benutzerfreundlichkeit und Betriebssicherheit weiter zu optimieren, so dass auch dem technisch weniger versierten Benutzer bzw. Patient das Gefühl vermittelt wird, die Betriebsweise des Geräts sicher zu beherrschen.

Diese Aufgabe wird erfindungsgemäß durch eine Vorrichtung mit den Merkmalen des Anspruchs 1 gelöst.

Wenn in diesem Sinne von Deaktivieren der Saugpumpe die Rede ist, so wird hierunter eine Betriebsunterbrechung des Geräts bis zu einem auf einfache Weise auszulösenden Neustart verstanden, das heißt, der gerade ablaufende Druckregelbetrieb und damit die Ansteuerung der Pumpe werden dauerhaft unterbrochen, bis der Benutzer einen Neustart und damit einen neuen Betriebszyklus initiiert oder bis der Behälter, insbesondere ein neuer ungebrauchter Behälter in seiner bestimmungsgemäßen Montageposition angeordnet ist. Mit der vorliegenden Erfindung wurde festgestellt, dass gerade bei am Körper des Patienten tragbaren Vorrichtungen zur Unterdruckwundbehandlung die häufigste Ursache für eine Fehlbedienung darin besteht, dass der Behälter zur Aufnahme von Körperflüssigkeiten von dem ersten Gehäuseteil der Vorrichtung, in dem sich die Saugpumpe und die elektronischen Steuerkomponenten befinden, gelöst wird, so dass es in der Folge zu steuerungstechnisch undifferenzierten Zuständen kommt. Wenn beispielsweise ausgehend von einem gerade aktiven Unterdrucksteuerungs- bzw. Regelungsbetrieb der Behälter von dem ersten Gehäuseteil der tragbaren Vorrichtung gelöst wird, so kann dies zu der regelungstechnischen Konsequenz führen, dass die elektronische Steuereinrichtung die Saugpumpe in Richtung zunehmende Saugleistung ansteuert, was häufig mit einer Zunahme der Geräuschentwicklung verbunden ist und den Benutzer verunsichern kann und außerdem weitere Steuerungsmaßnahmen durch den Benutzer erforderlich machen kann. Diese Situation kann sich gleichermaßen dann stellen, wenn ein ganz oder schon weitgehend mit Flüssigkeit befüllter Behälter gegen einen neuen bewusst ausgetauscht wird.

Mit der erfindungsgemäßen Ausbildung der Vorrichtung wird hingegen sichergestellt, dass ausgehend von einem laufenden Unterdruckregelbetrieb die Unterdruck erzeugende Saugpumpe durch die elektronische Steuereinrichtung deaktiviert wird, sobald der Behälter abgenommen wird und dies über die optoelektronische Sensoreinrichtung im Zusammenwirken mit der elektronischen Steuereinrichtung festgestellt wird. Auf diese Weise stellen sich keine undefinierten Zustände ein, die im Zusammenwirken mit etwa eingeleiteten Steuerungsmaßnahmen zu einer Verwirrung des Benutzers führen könnten. Stattdessen kann sich der Benutzer daran gewöhnen, dass er nach einem Behälteraustausch die Vorrichtung in der gewohnten Weise durch eine ihm gewohnte Betätigung wieder erneut in Betrieb zu nehmen hat, wobei alternativ auch ein automatischer Neustart erfolgen kann, sobald der abgelöste oder ein neuer Behälter bestimmungsgemäß am ersten Gehäuseteil befestigt ist. Solchenfalls arbeitet vorzugsweise dieselbe Sensoreinrichtung gewissermaßen in umgekehrter Richtung, indem sie der Steuereinrichtung die Wiederanordnung des Behälters signalisiert, so dass diese einen Neustart des zuvor abgelaufenen Programms vornehmen kann.

Der vorliegenden Erfindung kommt in besonderem Maße Bedeutung beim mobilen Einsatz von tragbaren Vorrichtungen der hier in Rede stehenden Art zu, da mit einer solchen tragbaren Vorrichtung ausgestattete Patienten fernab von einer klinischen Einrichtung auf sich selbst gestellt sind und deshalb auch komplex arbeitende Vorrichtungen eine auf ein Minimum reduzierte Mannigfaltigkeit von Zuständen aufweisen sollten, die der Patient zum einen erfassen und auf die der Patient zum anderen durch eine Bedienungsmaßnahme zu reagieren hat. Wird dem technisch unversierten Benutzer oder Patienten jedoch stets derselbe Zustand, nämlich Betrieb deaktiviert, dargeboten wenn der Behälter gelöst oder abgenommen wird, auf den er dann in stets derselben Weise, nämlich durch erneute Inbetriebnahme bzw. durch Wiederbefestigen desselben oder eines frischen Behälters zu reagieren hat, so fällt ihm dies bedeutend leichter. Mit der erfindungsgemäßen Ausbildung wird daher die Bedienbarkeit der Vorrichtung insgesamt erleichtert und damit auch die Anfälligkeit für Fehlbedienungen verringert und somit die Betriebssicherheit insgesamt erhöht.

Die vorausgehende Erwähnung des Signals bedeutet lediglich ein Zusammenwirken zwischen der optoelektronisch wirkenden Sensoreinrichtung und der elektronischen Steuereinrichtung. Die Sensoreinrichtung kann also im einfachsten Fall einen Schalter umfassen, der beim Anordnen des Behälters in seiner bestimmungsgemäßen Montageposition am ersten Gehäuseteil betätigt und beim Abnehmen des Behälters wieder geöffnet wird oder umgekehrt. Das an die Sensoreinrichtung gegebene Signal besteht dann in einer Zustandsänderung, die von einem Sensoreingang aufgenommen und in der elektronischen Steuereinrichtung verarbeitet und im entsprechenden Sinn erkannt wird und die weiteren Steuerungsprozesse, nämlich zumindest die Deaktivierung der Saugpumpe, auslöst.

Bei der erfindungsgemäßen Ausführungsform ist die Sensoreinrichtung optoelektronisch wirkend ausgebildet werden, so dass die Anwesenheit oder Nichtanwesenheit des Behälters in seiner bestimmungsgemäßen Montageposition durch optoelektronische Mittel, also im weitesten Sinn optisch erfasst wird.

Es erweist sich weiter als vorteilhaft, wenn eine Ausgabe- oder Anzeigeeinrichtung zur Erzeugung eines Signals vorgesehen ist, welches einen abrupten Druckanstieg mit einer Druckänderungsrate oberhalb des Schwellwerts und somit die Deaktivierung der Saugpumpe visuell oder akustisch vermittelt. Auf diese Weise kann dem Benutzer oder Patienten vermittelt werden, dass er in gewohnter Weise, insbesondere nach Wiederbefestigung des Behälters oder nach Austausch mit einem neuen Behälter, die Vorrichtung wieder in Betrieb zu nehmen hat.

In Weiterentwicklung der Erfindung kann es sich als vorteilhaft erweisen, wenn eine Ausgabe- oder Anzeigeeinrichtung vorgesehen ist, welche zur drahtlosen Übermittlung eines Signals an einen externen Empfänger ausgebildet ist, welches den abrupten Druckanstieg mit einer Druckänderungsrate oberhalb des Schwellwerts und somit die Deaktivierung der Saugpumpe an einen externen Empfänger vermittelt. Bei dem externen Empfänger kann es sich beispielsweise um eine Stationszentrale in einer Klinik oder eine Pflegeleitstelle oder dergleichen handeln, so dass die Deaktivierung des Druckregelbetriebs bzw. der Saugpumpe dort mitgeteilt wird und von dort weitere Schritte, insbesondere bei pflegebedürftigen Patienten, eingeleitet werden können.

Wie schon erwähnt kann es sich als vorteilhaft erweisen, wenn die Sensoreinrichtung gewissermaßen in umgekehrter Richtung arbeitet und ein Wiederanbringen des Behälters erkennt, so dass dann automatisch die Wiederaufnahme des Druckregelbetriebs erfolgen kann. Nach diesem Erfindungsgedanken ist die Sensoreinrichtung und die elektronische Steuereinrichtung so ausgebildet sind, dass eine (Wieder)Anordnung des Behälters in seiner bestimmungsgemäßen Position am ersten Gehäuseteil festgestellt wird, und dass ausgehend von einer zuvor ausgeführten Deaktivierung der Saugpumpe der zuvor eingestellte Druckregelbetrieb solchenfalls automatisch wieder aufgenommen wird.

Die erfindungsgemäße Vorrichtung kann weiter ein durch die Steuereinrichtung steuerbares Belüftungsventil umfassen, welches über eine zusätzlich zur Saugleitung vorgesehene Belüftungsleitung mit dem Wundraum verbindbar ist, so dass der Wundraum mit Außenluft belüftet werden kann.

Die Erfindung erweist sich als besonders vorteilhaft bei einer Vorrichtung, die weiter gekennzeichnet ist durch eine durch die elektronische Steuereinrichtung steuerbare Einrichtung zum Zuführen einer Spülflüssigkeit oder eines sonstigen Fluids, welche über eine zusätzlich zur Saugleitung vorgesehene Spülleitung mit dem Wundraum verbindbar ist, so dass Spülflüssigkeit oder ein sonstiges Fluid, insbesondere mit therapeutisch wirkenden Bestandteilen, in den Wundraum zugeführt werden kann. Es erweist sich solchenfalls als besonders vorteilhaft, wenn die elektronische Steuereinrichtung weiter so ausgebildet ist, dass sie beim Deaktivieren der Saugpumpe auch die Einrichtung zum Zuführen einer Spülflüssigkeit oder eines sonstigen Fluids deaktiviert. Es erweist sich nämlich als wesentlich betriebssicherer, diese Einrichtung zum Zuführen einer Spülflüssigkeit während eines Behälterwechsels oder einer sonstigen Betriebsstörung zusammen mit der Saugpumpe bis zu einem Neustart des Geräts dauerhaft zu deaktivieren.

Es gibt gattungsgemäße Vorrichtungen zur Unterdruckwundbehandlung, etwa EP 777 504 A, bei denen über einen Füllstandssensor ermittelt werden soll, ob der Behälter voll ist und gegen einen neuen ausgetauscht werden muss; wird der Behälter als voll erkannt, so wird die Saugpumpe deaktiviert, das heißt, der Druckregelbetrieb unterbrochen. Dies wurde mit der vorliegenden Erfindung aber als nachteilig erkannt, weil gerade bei tragbaren Vorrichtungen im mobilen Betrieb eine Füllstandsüberwachung des Behälters der vorbekannten Art häufig Fehlinformationen liefert. Beispielsweise wird infolge von Bewegungen des Benutzers, etwa beim Bücken, kurzzeitig ein Behälter-voll-Zustand detektiert, der tatsächlich nicht vorliegt. Des weiteren führt eine Deaktivierung der Saugpumpe in der Regel viel zu früh zu einem Abbau des Unterdrucks. Häufig steht nämlich ein ungebrauchter Behälter nicht zur Verfügung oder derartige Pflegemaßnahmen müssen zurückgestellt werden. In Weiterbildung der vorliegenden Erfindung wird deshalb vorgeschlagen, dass die elektronische Steuereinrichtung so ausgebildet ist, dass sie bei zutreffender oder unzutreffender Erkennung eines Behälter-voll-Zustands die Saugpumpe nicht deaktiviert, sondern den vorgegebenen Unterdruckregelbetrieb fortsetzt. Auf diese Weise wird nämlich sichergestellt, dass der Unterdruckregelbetrieb nicht zu früh oder irrtümlich unterbrochen wird. Auch wenn mit Sicherheit festgestellt wird, dass tatsächlich ein Behälterwechsel angezeigt ist, erweist es sich als vorteilhaft, wenn über die elektronische Steuereinrichtung der normale Unterdruckregelbetrieb fortgesetzt wird, das heißt, die Saugpumpe in der steuerungstechnisch vorbestimmten Weise den Unterdruck auf den Behälter aufrechterhält. Sofern dann der Behälter von dem ersten Gehäuseteil, das heißt der Basis der Vorrichtung gelöst wird, wird die Saugpumpe dauerhaft deaktiviert.

Ungeachtet dessen erweist es sich als vorteilhaft, wenn die elektronische Steuereinrichtung so ausgebildet ist, dass sie bei einem Unterdruckanstieg, das heißt bei einer Abnahme des absoluten Drucks, in dem Leitungsabschnitt zwischen Behälter und Saugpumpe, der insbesondere auf eine zunehmende Befüllung des Behälters oder sonstige insbesondere vorübergehende undefinierte Zustände oder Störungen, insbesondere infolge der Bewegung des Benutzers, schließen lässt, die Saugpumpe nicht deaktiviert, d.h. dauerhaft stillsetzt, so dass sie neu gestartet werden muss, sondern den vorgegebenen Unterdruckregelbetrieb fortsetzt. Der Unterdruckregelbetrieb wird also nach diesem Erfindungsgedanken nur bei einer festgestellten Abnahme des Behälters von dem ersten Gehäuseteil der Vorrichtung deaktiviert. Im Anschluss an den vorhergehenden Erfindungsgedanken kann auch ein zunehmender Unterdruck (Abnahme des absoluten Drucks), insbesondere ein abrupt zunehmender Unterdruck auf einen Behälter-voll-Zustand schließen lassen, nämlich insbesondere dann, wenn ein zwar luftdurchlässiger nicht aber flüssigkeitsdurchlässiger Filter im Bereich eines Behälterausgangs zu dem Leitungsabschnitt zur Saugpumpe verwendet wird. Steigt der Flüssigkeitsstand im Behälter bis bzw. über diesen Filter, so blockiert dieser zunehmend und führt zu einem Unterdruckanstieg in dem Leitungsabschnitt zwischen Behälter und Saugpumpe, der wiederum durch den Drucksensor detektiert wird.

Gegenstand der Erfindung ist desweiteren ein Verfahren mit den Merkmalen des Anspruchs 10.

Weitere Merkmale, Einzelheiten und Vorteile der Erfindung ergeben sich aus den beigefügten Patentansprüchen und aus der zeichnerischen Darstellung und nachfolgenden Beschreibung einer bevorzugten Ausführungsform der Erfindung. In der Zeichnung zeigt:
Figuren 1 a bis e verschiedene Ansichten einer bevorzugten Ausführungsform einer am Körper tragbaren Vorrichtung zur Bereitstellung von Unterdruck für medizinische Anwendungen;
Figuren 2a bis e verschiedene Ansichten eines eine Unterdruck erzeugende Einrichtung und Steuerungskomponenten umfassenden ersten Gehäuseteils der Vorrichtung nach Figur 1;
Figuren 3a bis i verschiedene Ansichten eines einen Behälter zur Aufnahme von Körperflüssigkeiten bildenden zweiten Gehäuseteils der Vorrichtung nach Figur 1;
Figuren 4a bis e den Figuren 1a bis e entsprechende Ansichten einer weiteren Ausführungsform einer Vorrichtung, wobei der zweite Gehäuseteil größer dimensioniert ist als bei der Vorrichtung nach Figuren 1a bis e; und
Figur 5 eine Schnittansicht durch die Vorrichtung im Bereich der Unterdruckkommunikation zwischen dem ersten und zweiten Gehäuseteil;
Figuren 6 eine schematische Darstellung des ersten und zweiten Gehäuseteils mit Andeutung der steuerungstechnischen Komponenten.

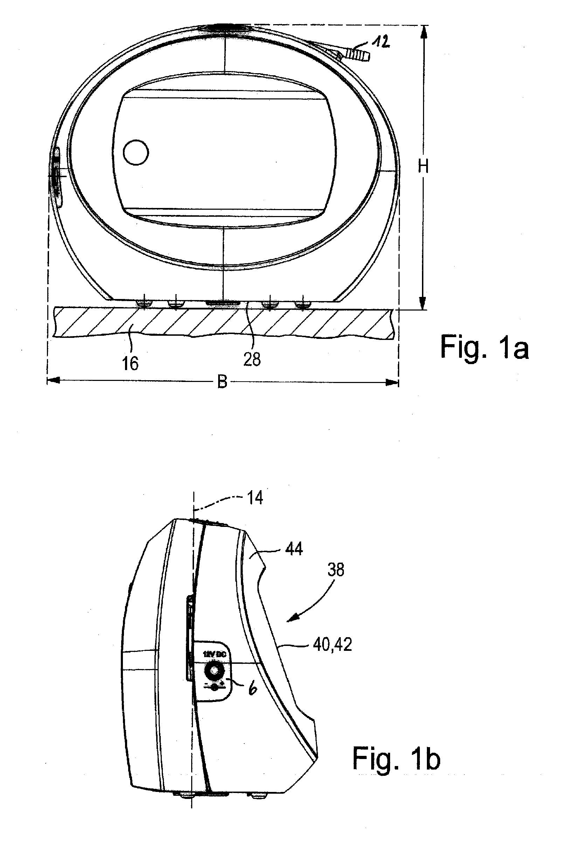

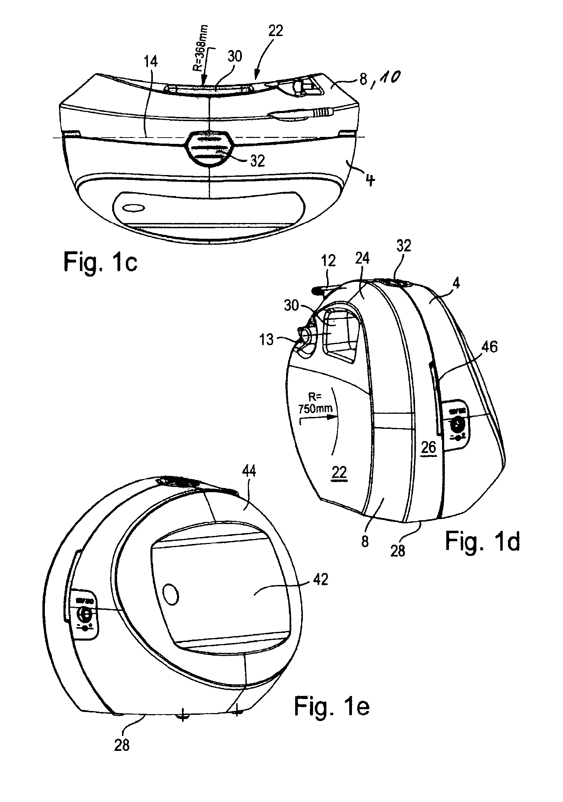

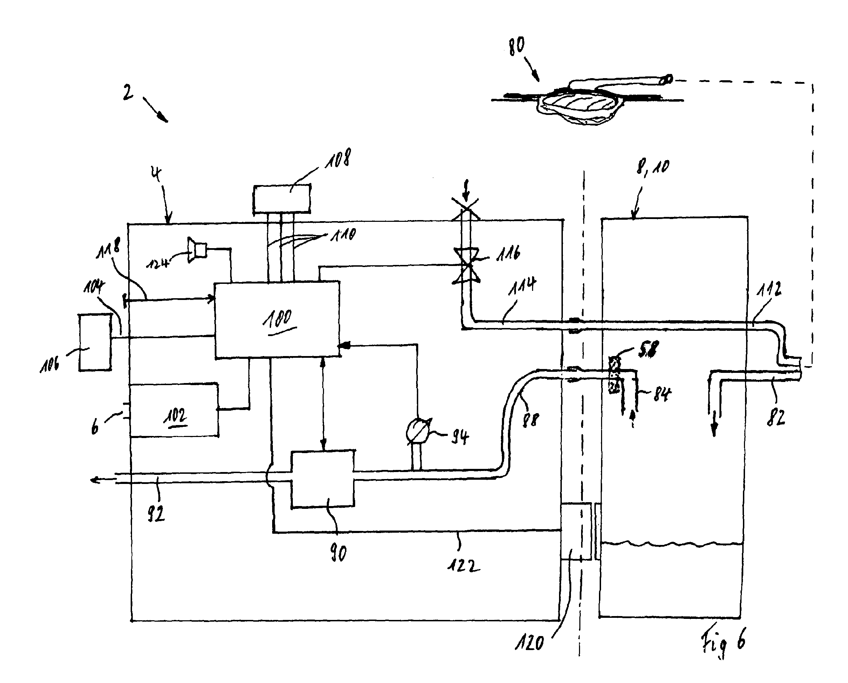

Zunächst werden anhand der Figuren 1 - 5 zwei Ausführungsformen einer tragbaren Vorrichtung 2 zur Bereitstellung von Unterdruck für medizinische Anwendungen beschrieben, die sich aber nur hinsichtlich der Dimensionierung und Ausbildung eines noch zu beschreibenden Behälters zur Aufnahme von Körperflüssigkeiten unterscheiden. Danach wird anhand der Figur 6 die erfindungsgemäße Ausbildung der steuerungstechnischen Komponenten der tragbaren Vorrichtung 2 beschrieben.

Die Figuren 1a bis e zeigen eine erste Ausführungsform einer tragbaren Vorrichtung 2 zur Bereitstellung von Unterdruck für medizinische Anwendungen. Die Vorrichtung umfasst einen ersten Gehäuseteil 4, in dem eine Unterdruck erzeugende Einrichtung in Form einer in Figur 6 dargestellten Luftsaugpumpe 90 sowie elektrische und elektronische Steuerkomponenten für die Vorrichtung insgesamt aufgenommen sind, einschließlich Batterien oder vorzugsweise wiederaufladbare Akkus. Ein Ladeanschluss für die Akkus ist mit Bezugszeichen 6 bezeichnet. Des Weiteren umfasst die Vorrichtung 2 einen zweiten Gehäuseteil 8, welcher einen Behälter 10 zur Aufnahme von Körperflüssigkeiten, insbesondere zur Aufnahme von aus einer Wunde abgesaugten Wundsekreten, bildet. Bevorzugtermaßen ist der gesamte zweite Gehäuseteil 8 als wegwerfbarer Einmalartikel ausgebildet. An seinem oberen Bereich ist ein Anschlussstutzen 12 für eine erst in Figur 6 dargestellte Saugleitung 82 vorgesehen, die dann beispielsweise bei der Verwendung der Vorrichtung 2 zur Unterdrucktherapie von Wunden zu einem die Wunde druckdicht verschließenden Wundverband 80 führt und dort beispielsweise über einen Port mit dem Wundraum kommuniziert, um im Wundraum einen Unterdruck anzulegen und aufrechtzuerhalten und Wundsekrete in den Behälter 10 abzusaugen. Hierfür kommuniziert der Behälter 10 mit der Saugpumpe. Weiter ist ein Anschluss 13 für einen optionalen Mess- oder Spülkanal, der wie die Saugleitung zur Wunde führt, dargestellt. Dieser Anschluss durchgreift den zweiten Gehäuseteil 8 und mündet in den ersten Gehäuseteil 4, von wo aus der Mess- oder Spülkanal beispielsweise mit Luft als Spülmedium beaufschlagt werden kann und/oder ein Druck in diesem Mess- oder Spülkanal detektiert und ausgewertet werden kann.

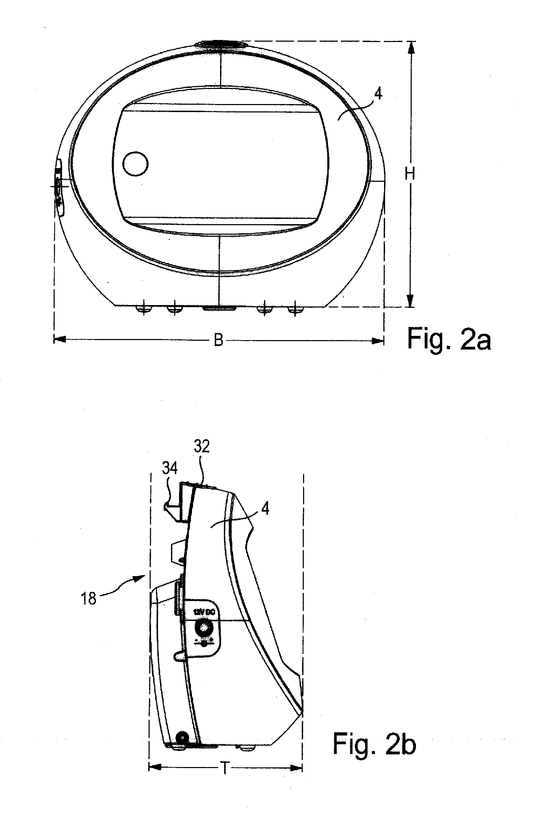

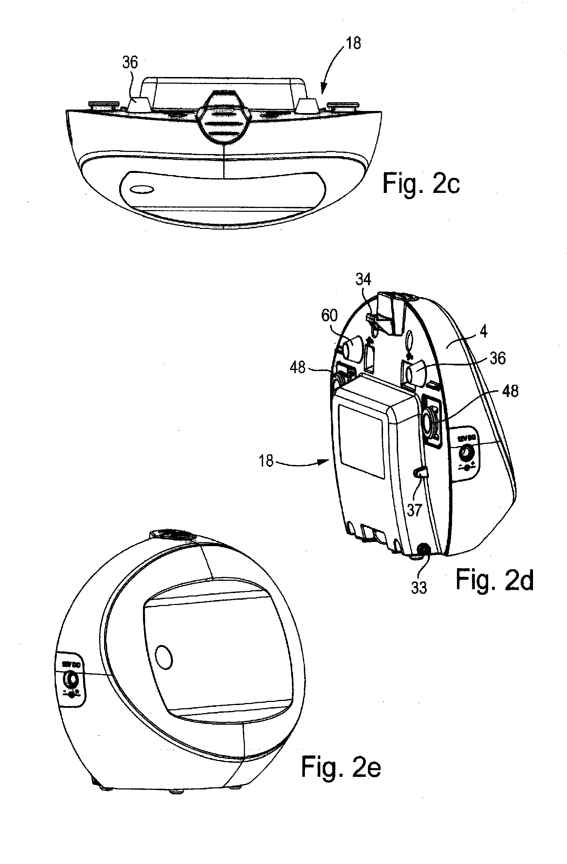

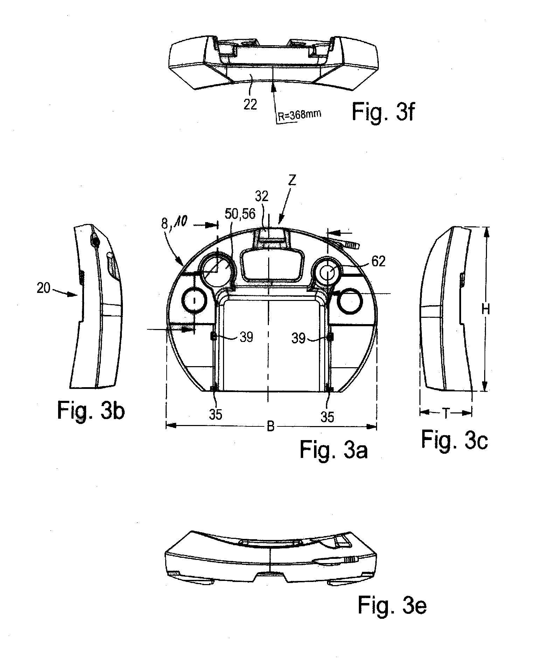

Im bevorzugten dargestellten Fall liegen die Gehäuseteile 4 und 8 über eine im Wesentlichen vertikale Trennebene 14, die in verschiedenen Figuren angedeutet ist, gegeneinander an. Wenn die Vorrichtung 2, wie in Figur 1a angedeutet, auf einer ebenen horizontalen Unterlage 16 abgestellt ist, so ist die Trennebene 14 im Wesentlichen vertikal orientiert. Dies bedeutet, dass die beiden Gehäuseteile 4, 8 nicht ineinander eingesetzt oder übereinander gestapelt sind, sondern dass sie nebeneinander im bestimmungsgemäß zusammengesetzten Zustand der Vorrichtung 2 zu liegen kommen. Der Begriff der Trennebene 14 ist also nicht in dem Sinn zu verstehen, dass es sich um eine geometrisch ebene Fläche handeln muss, was unmittelbar aus den Figuren 2a bis e ersichtlich wird, welche den ersten Gehäuseteil 4 in verschiedenen Ansichten zeigen. Man erkennt sofort, dass die dem zweiten Gehäuseteil 8 zugewandte Seite 18 des ersten Gehäuseteils 4 keinesfalls eben, sondern mit einer Vielzahl von in Richtung auf den zweiten Gehäuseteil 8 vorspringenden Elementen ausgebildet ist. Die dem ersten Gehäuseteil 4 zugewandte Seite 20 des zweiten Gehäuseteils 8 ist im Wesentlichen komplementär zu der Ausbildung der Seite 18 des ersten Gehäuseteils 4 ausgebildet, so dass die beiden Gehäuseteile 4, 8 nur in einer korrekten Weise miteinander gefügt bzw. aneinander befestigt werden können. Die beiden Gehäuseteile 4, 8 sind insgesamt scheibenförmig ausgebildet, d. h. ihre Breite B in horizontaler Richtung und ihre Höhe H in vertikaler Richtung sind jeweils größer als ihre Tiefe T in horizontaler Richtung und senkrecht zur Breitenerstreckung. Hierdurch ist es möglich, dass die Vorrichtung 2 in Tiefenrichtung insgesamt so ausgebildet und bemessen werden kann, dass sie bequem am Körper eines Benutzers getragen werden kann. Erfindungsgemäß ist die Vorrichtung 2 so ausgebildet, dass die nebeneinander angeordneten Behälterteile 4, 8 derart am Körper positionierbar sind, dass der zweite Behälterteil 8 körperzugewandt, also zwischen dem Körper und dem ersten Gehäuseteil 4, zu liegen kommt und der erste Gehäuseteil 4 körperabgewandt zu liegen kommt, also im Wesentlichen die Sichtseite der Vorrichtung 2 bildet. Daher ist die dem Körper des Benutzers zugewandte Seite 22 des zweiten Gehäuseteils 8 verrundet ausgebildet. Wie sich den Figuren 1c, 1 d, 3f, 3e entnehmen lässt, ist die körperzugewandte Seite 22 im Schnitt mit einer horizontalen Ebene betrachtet konkav ausgebildet und umfasst im beispielhaft dargestellten Fall abschnittsweise einen Krümmungsradius R von beispielhaft 368 mm (Figur 1c, 3f). Zusätzlich ist die körperzugewandte Seite 22 im Schnitt mit einer vertikalen Ebene betrachtet ebenfalls konkav ausgebildet und weist dort einen Krümmungsradius R von beispielhaft 750 mm auf (Figur 1d). Auf diese Weise lässt sich die Vorrichtung 2 ergonomisch im Hüftbereich eines Benutzers anordnen und tragen.

Man erkennt des Weiteren, dass der zweite Gehäuseteil 8 auf seiner körperzugewandten Seite 22 in einem oberen Bereich und auch seitlich eine Abschrägung 24 weg vom Körper des Benutzers in Richtung auf den ersten Gehäuseteil 4 bzw. in Richtung auf Seitenwandungen 26 oder eine Umfangsstirnseite der Scheibenform des zweiten Gehäuseteils 8 hin umfasst. Die Abschrägung 24 ist im beispielhaft dargestellten Fall umlaufend ausgebildet; sie erstreckt sich ausgehend von der Standseite 28 von unten nach oben, verläuft dort bogenförmig zur anderen Seite und dann wieder nach unten zur Standseite 28 zurück.

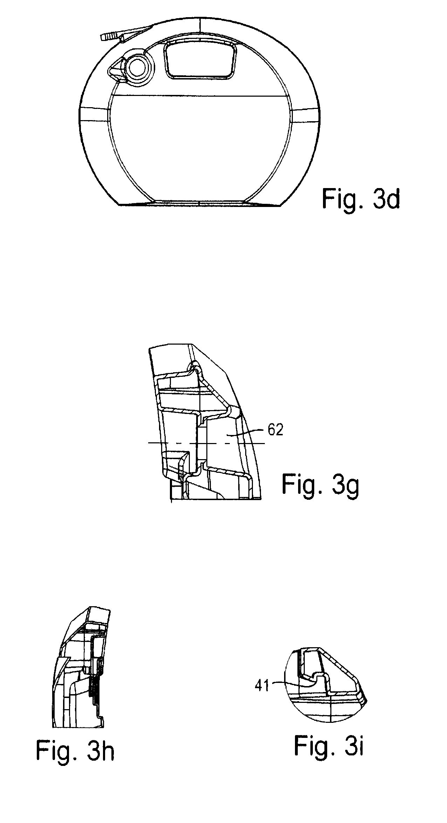

Man erkennt des Weiteren aus den Figuren 1d und 3, dass auf der körperzugewandten Seite 22 des zweiten Gehäuseteils 8 eine Eingriffsvertiefung 30 in Form einer durch den zweiten Gehäuseteil 8 hindurch gehenden Öffnung ausgebildet ist, und zwar in einem oberen leicht vom Körper weg geneigten Bereich des zweiten Gehäuseteils 8. Hierdurch kann die Vorrichtung 2 insgesamt oder nur deren zweiter Gehäuseteil 8 mit einer Hand ergriffen und gehandhabt werden.

Bei der dargestellten bevorzugten Ausführungsform ist nahe bei dieser Eingriffsvertiefung 30 in einer Oberseite der Vorrichtung 2 ein manuell bedienbares Betätigungsorgan 32, beispielsweise in Form eines Tasters, vorgesehen, der auf ein Verriegelungs- oder Hintergriffsmittel 34 einwirkt (s. Figuren 2b und 2d). Im gefügten Zustand der beiden Gehäuseteile 4 und 8 befindet sich das Verriegelungs- oder Hintergriffsmittel 34 in einem die beiden Gehäuseteile 4, 8 formschlüssig aneinander haltenden verriegelten Zustand. Erst durch Betätigen des Betätigungsorgans 32 wird die Verriegelung gelöst, so dass die Gehäuseteile 4, 8 voneinander separiert werden können. Durch die Anordnung und Ausbildung der Eingriffsvertiefung 30 und des manuell bedienbaren Betätigungsorgans 32 in räumlicher Nähe zueinander und derart, dass ein Benutzer sowohl in die Eingriffsvertiefung 30 eingreifen als auch mit einem Finger derselben Hand gleichzeitig das Betätigungsorgan 32 zu bedienen vermag, wird eine Einhandbedienung zum Lösen des zweiten Gehäuseteils 8 vom ersten Gehäuseteil 4 realisiert. Dies erweist sich als besonders vorteilhaft, da solchenfalls ein mit Körperflüssigkeiten befüllter zweiter Gehäuseteil 8 mit nur einer Hand gelöst und in ein Entsorgungsbehältnis gegeben werden kann.

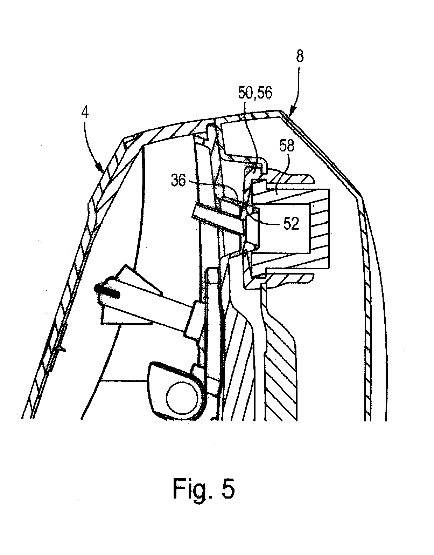

Zum Fügen der beiden Gehäuseteile 4, 8 wird der zweite Gehäuseteil 8 leicht schräg von hinten und von oben mit seinem unteren Rand auf zwei einen Drehpunkt bildende Zapfen 33 (Figur 2d) des ersten Gehäuseteils 4 aufgesetzt. In dem zweiten Gehäuseteil ist hierfür am unteren Rand ein ausgesparter Bereich 35 (Figur 3a) zur Aufnahme des Zapfens 33 ausgebildet. Wenn Zapfen 33 und ausgesparter Bereich 35 miteinander in Eingriff sind, so lässt sich der zweite Gehäuseteil 8 gegen den ersten Gehäuseteil 4 schwenken. Hierdurch werden die einander zugewandten Seiten 18, 20 gegeneinander gelegt und gelangen so selbstzentrierend (unterstützt durch weitere Führungsoder Zentriermittel 37 (Figur 2d) und 39 (Figur 3a) und die komplementäre Ausbildung der einander zugewandten Seiten 18, 20 der Gehäuseteile 4,8 in ihre bestimmungsgemäße Position. Durch Gegeneinanderbewegen der beiden Gehäuseteile 4, 8, insbesondere im Wesentlichen quer zu der vertikalen Trennebene 14, wird das Verriegelungs- oder Hintergriffsmittel 34 selbsttätig ausgelenkt und rastet dann in seine die Gehäuseteile 4, 8 gegeneinander verriegelnde Stellung. Hierfür ist an dem zweiten Gehäuseteil 8 ein Rasthaken 41 (Figur 3i) vorgesehen, der von dem Verriegelungs- oder Hintergriffsmittel 34 untergriffen wird. Wenn die Gehäuseteile 4, 8 in ihre verriegelte Stellung gebracht werden, wird dann auch automatisch eine Unterdruckkommunikation zwischen dem Inneren des Behälters 10 des zweiten Gehäuseteils 8 und der Unterdruck erzeugenden Einrichtung über Anschlussmittel 36 hergestellt (später im Zusammenhang mit Figur 5 beschrieben).

Eine körperabgewandte Sichtseite 38 des ersten Gehäuseteils 4 ist leicht zur Vertikalen geneigt ausgebildet, so dass sich die Scheibenform nach oben hin verjüngt. Auf diese Weise ist eine leichtere Einsichtnahme auf die Sichtseite 38 gegeben. Dort sind Bedienelemente 40 und Anzeigeelemente 42 insbesondere in Form eines Touchscreens mit einer Schaltfolie vorgesehen. Im Wesentlichen die gesamte Sichtseite 38 ist von einer flächenhaften Abdeckung 44 überfangen oder gebildet, so dass im Bereich der Bedienelemente 40 keine Schmutz aufnehmenden Fugen gebildet werden.

Des Weiteren zeigen die Figuren im Bereich der Trennebene 14 zwischen den gegeneinander anliegenden Gehäuseteilen 4, 8 einen Einsteckschlitz 46 zum Einstecken und lösbaren Fixieren eines Befestigungsmittels, insbesondere und vorzugsweise in Form eines flexiblen Gürtels, oder eines Bügels oder einer Lasche, an dem/der beispielsweise ein Gürtel oder ein Schultertragriemen befestigt werden kann, oder in sonstiger Form. Es erweist sich als vorteilhaft, dass dieses Befestigungsmittel von den Gehäuseteilen 4, 8 gelöst werden kann und somit nicht stört, wenn die Vorrichtung 2 im stationären Betrieb, also auf einer vorzugsweise ebenen Unterlage 16 stehend, benutzt wird, etwa wenn ein hiermit zu behandelnder Patient in einem Krankenbett ruht. In Figur 2d sind an der Seite 18 des ersten Gehäuseteils 4 Mittel 48 angedeutet, an denen die in den Einsteckschlitz 46 eingesteckten Befestigungsmittel festlegbar oder gehalten sind.

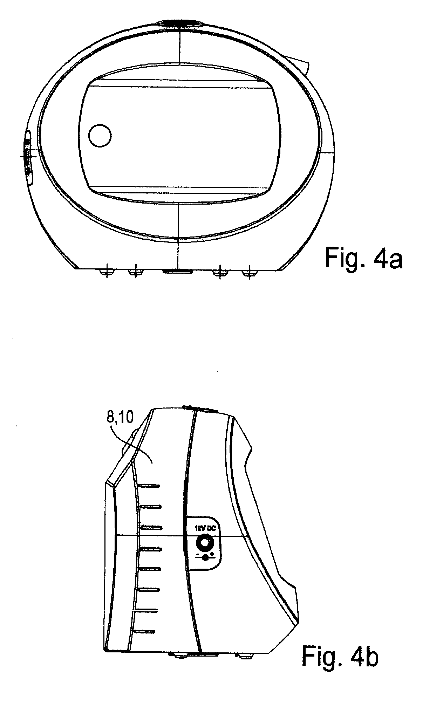

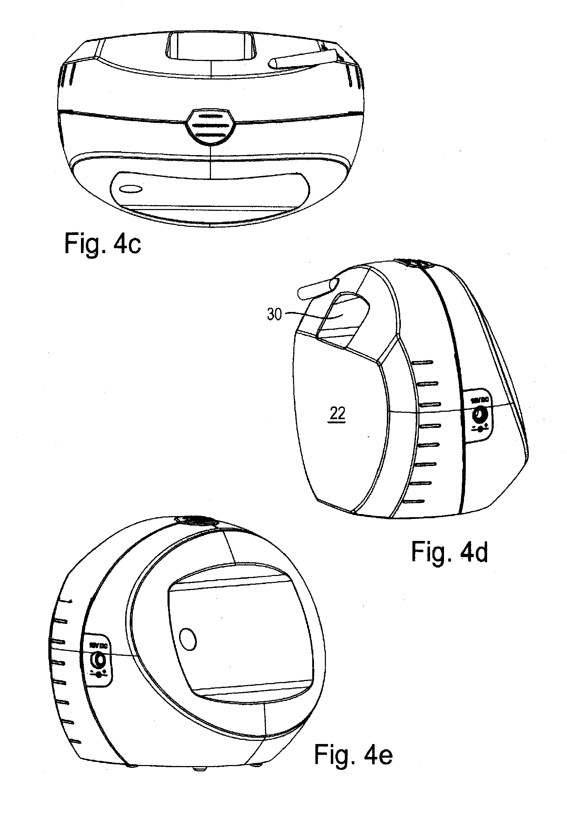

Die in Figuren 4a bis e dargestellte weitere Ausführungsform der erfindungsgemäßen Vorrichtung unterscheidet sich von der in Figuren 3 dargestellten Ausführungsform dadurch, dass der zweite Gehäuseteil 8 und der von ihm gebildete Behälter 10 großvolumiger ausgebildet ist. Die Abschrägung im oberen Bereich der körperzugewandten Seite 22 des zweiten Gehäuseteils 8, wo die Griffmulde 30 ausgebildet ist, ist noch etwas stärker vom Körper des Benutzers weg geneigt. Auf diese Weise ist ein noch besserer Zugriff möglich. Dieser größere zweite Gehäuseteil 8 eignet sich eher für einen stationären Betrieb der Vorrichtung 2; er könnte hierfür auch eine nach außen konvex gewölbte Seite 22 aufweisen oder gar noch ausladender ausgebildet sein als dies die Figuren 4 zeigen.

Figur 5 zeigt im Einzelnen die Ausbildung der Unterdruckkommunikation zwischen dem Inneren des den Behälter 10 bildenden zweiten Gehäuseteils 8 und dem ersten Gehäuseteil 4. Die Ansaugseite einer nicht dargestellten Unterdruck erzeugenden Einrichtung führt zu dem konisch ausgebildeten Anschlussmittel 36, welches sich konisch in Richtung auf den zweiten Gehäuseteil 8 verjüngt. Auf diese Weise kann gegen das konische Anschlussmittel 36 des ersten Gehäuseteils 4 ein wenigstens geringfügig nachgiebig ausgebildetes Gegenanschlussmittel 50 des zweiten Gehäuseteils 8 dichtend angelegt werden, welches im beispielhaft dargestellten Fall eine kreisförmige Öffnung 52 aufweist, die von einer nachgiebigen Dichtlippe 54 begrenzt ist. Dieses Gegenanschlussmittel 50 mündet in das Innere des zweiten Gehäuseteils 8. Es bildet zugleich ein Filteraufnahmemittel 56 für einen Filter 58, der im beispielhaft dargestellten Fall als topfförmiger Filter ausgebildet ist und verhindert, dass Bakterien in den ersten Gehäuseteil 4 eingesogen werden. Man erkennt ohne weiteres, dass beim Gegeneinanderbewegen der beiden Gehäuseteile 4, 8 das Anschlussmittel 36 des ersten Gehäuseteils 4 mit dem Gegenanschlussmittel 50 des zweiten Gehäuseteils 8 eine nach außen abgedichtete Druckkommunikation ausbildet.

In ähnlicher Weise ist die Kopplung zwischen dem Anschluss 13 für einen Mess- bzw. Spülkanal und dem zugehörigen ebenfalls beispielhaft konusförmig ausgebildeten Anschlussmittel 60 am ersten Gehäuseteil 4 ausgebildet. Wie aus Figur 3g ersichtlich ist, lässt sich in die Durchgangsöffnung 62 in dem zweiten Gehäuseteil 8 ein nicht dargestelltes Kopplungs- oder Tüllenteil einsetzen, welches dann den in Figur 1d dargestellten Anschluss 13 für den Mess- oder Spülkanal bildet. Dieses nicht dargestellte Kopplungs- oder Tüllenteil kann dann mit dem konusförmigen Anschlussmittel 60 druckdicht gekoppelt werden. Auf diese Weise kann über eine Leitung ein fluides Medium, insbesondere Luft oder eine Spülflüssigkeit, zur Wunde geleitet werden, um die Absaugung von Wundsekreten zu unterstützen. Typischerweise sind eine Mess- oder Spülleitung und die Saugleitung als wegwerfbare Einwegkomponenten Zubehörbestandteil zu dem zweiten Gehäuseteil; sie werden nach Gebrauch zusammen mit diesem entsorgt.

Figur 6 zeigt die vorausgehend beschriebene oder eine ähnliche Vorrichtung 2 zur Bereitstellung von Unterdruck für medizinische Anwendungen in rein schematischer Darstellung, wobei für entsprechende Bauelemente entsprechende Bezugszeichen verwendet werden. In Figur 6 sind jedoch nur die für die nachfolgende Beschreibung der Funktionsweise relevanten Komponenten dargestellt. Man erkennt schematisch eine in Figur 6 lediglich angedeutete mit Unterdruck zu therapierende Wunde mit einer unterdruckdichten Wundauflage 80, zu der die aus dem Behälter 10 ausmündende Saugleitung 82 führt. Aus dem Behälter 10 führt ein weiterer Leitungsabschnitt 84 durch den schon erwähnten Filter 58 hindurch nach außen. Wenn der Behälter 10 bzw. der erste Gehäuseteil 8 in seine Betriebsposition an dem ersten oder Basisgehäuseteil 4 der Vorrichtung 2 gebracht wird, so wird der Leitungsabschnitt 84 mit einem weiteren Leitungsabschnitt 88 innerhalb des ersten Gehäuseteils gekoppelt, welcher zu der Ansaugseite der Saugpumpe 90 führt. Dies wurde vorausgehend nur beispielhaft anhand der Figur 5 beschrieben. Im Betrieb der Saugpumpe 90 wird somit über die Leitungsabschnitte 88, 84 ein Unterdruck an den Behälter 10 und an die Saugleitung 82 gelegt und die von dort angesaugte Luft über eine Ausblasleitung 92 an die Umgebung ausgestoßen, wobei zusätzlich nicht dargestellte Schalldämpferelemente und gegebenenfalls weitere Filter vorgesehen sein können.

Des weiteren ist ein Drucksensor 94 zur Messung des Drucks in dem Leitungsabschnitt 88 zwischen Behälter 10 und Saugpumpe 90 vorgesehen. Dessen Signale werden an eine insgesamt mit dem Bezugszeichen 100 bezeichnete programmierbare elektronische Steuereinrichtung gegeben, welche die Vorrichtung 2 insgesamt steuert bzw. regelt. Des weiteren dargestellt ist der eingangs schon erwähnte Ladeanschluss 6 für Akkus, die in einem Kompartiment 102 untergebracht sind, sowie ein Anschluss 104 für ein schematisch angedeutetes Netzteil 106. Mit Bezugszeichen 108 ist eine Display-Einheit mit einer vorzugsweise vorgesehenen kapazitiven Schaltfolie bezeichnet, über welche die Bedienung der Vorrichtung insgesamt ausführbar ist. Die elektrische Verbindung zu der elektronischen Steuereinrichtung 100 ist über elektrische Leitungen 110 nur angedeutet. Durch die elektronische Steuereinrichtung 100 erfolgt die Ansteuerung der Saugpumpe 90, indem mittels der Signale des Drucksensors 94 eine Druck- bzw. Unterdruckregelung durch an sich bekannte Steuer- und Regelmechanismen (Soll-/Ist-Regelmechanismen) ausgeführt wird, so dass in dem Leitungsabschnitt 88 der dem gerade gewählten Programm entsprechende Druckwert eingeregelt wird.

Des weiteren dargestellt ist eine zusätzliche Spül- oder Belüftungsleitung 112, die nur im beispielhaft dargestellten Fall durch den Behälter 10 hindurchführt und ebenso wie die Saugleitung 82 zu der Wundabdeckung 80 führt. Beim Anbringen des Behälters 10 in seiner bestimmungsgemäßen Montageposition am ersten Gehäuseteil 4 kommuniziert diese Spülleitung 112 mit einem im ersten Gehäuseteil 4 vorgesehenen Leitungsabschnitt 114, in dem ein elektromagnetisch betätigtes Ventil 116 vorgesehen ist, welches durch die elektronische Steuereinrichtung 100 betätigbar ist und im geöffneten Zustand den Leitungsabschnitt 114 mit der Atmosphärenluft verbindet, so dass ein Luftstrom über die Spülleitung 112 in Richtung Wunde erzeugt werden kann.

Die Vorrichtung 2 und deren elektronische Steuereinrichtung 100 verfügen auch über eine Datenschnittstelle 118, vorzugsweise eine USB-Schnittstelle, mittels derer die elektronische Steuereinrichtung 100 bzw. deren Betriebsweise programmiert werden kann.

Weiter zeigt Figur 6 die erfindungsgemäße Sensoreinrichtung 120, die über lediglich angedeutete Betriebs- bzw. Signalleitungsmittel 122 mit der elektronischen Steuereinrichtung 100 zusammenwirkt. Diese Sensoreinrichtung 120 kann in vielfältiger Weise ausgebildet sein. Mithilfe der Sensoreinrichtung 120 kann festgestellt werden, wenn während des laufenden Druckregelbetriebs der Behälter 10 aus seiner bestimmungsgemäßen Montageposition am ersten Gehäuseteil 4 entfernt wird. Hierdurch ändert sich im weitesten Sinne der Zustand der Sensoreinrichtung 120, was durch die elektronische Steuereinrichtung 100 festgestellt werden kann, woraufhin die Steuereinrichtung 100 dann unmittelbar die Saugpumpe 90 deaktiviert.

Gleichzeitig kann die Sensoreinrichtung 120 aber auch dazu verwendet werden, das Wiederanbringen des Behälters oder das Anbringen eines frischen Behälters zu detektieren, und zwar sowohl vor Beginn eines Druckregelbetriebs oder nach einer Deaktivierung, d. h. Unterbrechung des Druckregelbetriebs. Diese Zustandsänderung der Sensoreinrichtung 120 wird dann durch die elektronische Steuereinrichtung festgestellt, die daraufhin automatisiert die Wiederinbetriebnahme des zuvor unterbrochenen Druckregelbetriebs veranlassen kann, wenn dies programmtechnisch vorgesehen ist, wobei dann vorzugsweise dieselben Betriebsparameter Druckregelbetriebs verwendet werden. Es wäre aber auch denkbar, dass das Wiederanbringen des Behälters steuerungstechnisch nur als Betriebsfreigabezustand behandelt wird, so dass der Benutzer selbst einen Neustart des Druckregelbetriebs initiieren muss.

Auf die erfindungsgemäße Weise wird ein undifferenzierter Zustand infolge Ablösens des Behälters und insbesondere ein Hochsteuern der Saugpumpe vermieden. Es wird somit mit definierten Zuständen gearbeitet, und der Benutzer wird daran gewöhnt, dass er bei einem beabsichtigten oder unbeabsichtigten Lösen des Behälters einen Neustart des Systems durchzuführen hat oder dies automatisiert durch bestimmungsgemäßes Befestigen des Behälters erfolgt.

Des weiteren erweist es sich als besonders vorteilhaft, dass die elektronische Steuereinrichtung 100 bei einem Unterdruckanstieg in dem Leitungsabschnitt 88 zwischen Behälter 10 und Saugpumpe 90, der beispielsweise auf eine zunehmende Befüllung des Behälters schließen lässt, die Saugpumpe nicht deaktiviert, sondern den vorgegebenen Unterdruckregelbetrieb fortsetzt. Beispielsweise ist dieser Unterdruckanstieg, das heißt die Abnahme des absoluten Drucks, nur darauf zurückzuführen, dass kurzzeitig aufgrund einer Neigung des Behälters 10 durch die Mobilität des Benutzers der flüssigkeitsdichte Filter 58 blockiert, ohne dass der Behälter 10 schon derart befüllt ist, dass ein Behälterwechsel angezeigt ist, oder es liegt eine andere nur kurzzeitige Blockierung oder Abklemmung eines Leitungsmittels vor. Doch auch ungeachtet dessen erweist es sich als vorteilhaft, dass eine Deaktivierung des Druckregelbetriebs und der Saugpumpe 90 durch Störbetrieb nur dann erfolgt, wenn ein abrupter Druckanstieg, also abrupt abnehmender Unterdruck, in dem Leitungsabschnitt 88 zwischen Behälter 10 und Saugpumpe 90 festgestellt wird, was durch Ermittlung und Vergleich der Druckänderungsrate Δ p/Δ t in Richtung abnehmenden Unterdrucks mit einem vorgegebenen Schwellwert festgestellt wird.

## Patentansprüche

1. Am Körper eines Benutzers tragbare Vorrichtung (2) zur Bereitstellung von Unterdruck zur medizinischen Unterdruckbehandlung von Wunden am menschlichen oder tierischen Körper, umfassend einen ersten Gehäuseteil (4) mit einer unterdruckerzeugenden Saugpumpe (90) in dem ersten Gehäuseteil (4), einem zweiten Gehäuseteil (8), welcher einen Behälter (10) zur Aufnahme von Körperflüssigkeiten, insbesondere von aus einer Wunde abgesaugten Wundsekreten, bildet, wobei der den Behälter (10) bildende zweite Gehäuseteil (8) lösbar an dem ersten Gehäuseteil (4) der Vorrichtung befestigbar ist und im befestigten Zustand von der Saugpumpe (90) mit Unterdruck beaufschlagbar ist, wobei die Gehäuseteile (4, 8) über eine im wesentlichen vertikale Trennebene (14) gegeneinander anliegen und dabei im bestimmungsgemäß zusammengesetzten Zustand der Vorrichtung (2) nebeneinander zu liegen kommen, wobei die beiden Gehäuseteile (4, 8) insgesamt scheibenförmig ausgebildet sind, d. h. ihre Breite (B) in horizontaler Richtung und ihre Höhe (H) in vertikaler Richtung jeweils größer sind als ihre Tiefe (T) in horizontaler Richtung und senkrecht zur Breitenerstreckung, so dass die nebeneinander angeordneten Gehäuseteile (4, 8) derart am Körper positionierbar sind, dass der zweite Gehäuseteil (8) körperzugewandt, also zwischen dem Körper und dem ersten Gehäuseteil (4), zu liegen kommt und der erste Gehäuseteil (4) körperabgewandt zu liegen kommt, also im Wesentlichen die Sichtseite der Vorrichtung (2) bildet, und wobei am Behälter (10) ein Anschluss (12) für eine zum Körper führende Saugleitung (82) vorgesehen ist, so dass eine Unterdruckkommunikation zwischen der Saugpumpe (90), dem Behälter (10) und der zum Körper führenden Saugleitung (82) herstellbar ist, eine programmierbare elektronische Steuereinrichtung (100), welche mindestens unter Berücksichtigung von vorgegebenen und/oder vorgebbaren Parametern und von durch den Drucksensor (94) gemessenen Druckwerten die Saugpumpe (90) ansteuern kann, wobei eine optoelektronisch wirkende Sensoreinrichtung (120) vorgesehen ist, mittels derer durch die elektronische Steuereinrichtung (100) feststellbar ist, wenn der Behälter (10) während des Unterdruckregelbetriebs von dem ersten Gehäuseteil (4) abgenommen wird, wobei die Sensoreinrichtung (120) ein Signal ausgibt, anhand dessen die elektronische Steuereinrichtung (100) die Abnahme des Behälters (10) erkennt, und dass die elektronische Steuereinrichtung (100) so ausgebildet ist, dass sie die Saugpumpe (90) dann deaktiviert.

2. Vorrichtung nachAnspruch 1, **dadurch gekennzeichnet, dass** eine Ausgabeoder Anzeigeeinrichtung (108) zur Erzeugung eines Signals vorgesehen ist, welches die Abnahme des Behälters visuell oder akustisch vermittelt.

3. Vorrichtung nach einem oder mehreren der vorstehenden Ansprüche, **dadurch gekennzeichnet, dass** eine Ausgabe- oder Anzeigeeinrichtung vorgesehen ist, welche zur drahtlosen Übermittlung eines Signals an einen externen Empfänger ausgebildet ist, welches die Abnahme des Behälters an einen externen Empfänger vermittelt.

4. Vorrichtung nach einem oder mehreren der vorstehenden Ansprüche, **dadurch gekennzeichnet, dass** die Sensoreinrichtung (120) und die elektronische Steuereinrichtung (100) so ausgebildet sind, dass eine Anordnung des Behälters (10) in seiner bestimmungsgemäßen Position am ersten Gehäuseteil (4) festgestellt wird, und dass ausgehend von einer zuvor ausgeführten Deaktivierung der Saugpumpe (90) der zuvor eingestellte Druckregelbetrieb dann wieder aufgenommen wird.

5. Vorrichtung nach einem oder mehreren der vorstehenden Ansprüche, **gekennzeichnet durch** ein **durch** die elektronische Steuereinrichtung (100) steuerbares Belüftungsventil (116), welches über eine zusätzlich zur Saugleitung vorgesehene Belüftungsleitung (112) mit dem Wundraum verbindbar ist, sodass der Wundraum mit Außenluft belüftet werden kann.

6. Vorrichtung nach einem oder mehreren der vorstehenden Ansprüche, **gekennzeichnet durch** eine **durch** die elektronische Steuereinrichtung (100) steuerbare Einrichtung zum Zuführen einer Spülflüssigkeit oder eines sonstigen Fluids, welche über eine zusätzlich zur Saugleitung vorgesehene Spülleitung mit dem Wundraum verbindbar ist, so dass Spülflüssigkeit oder ein sonstiges Fluid in den Wundraum zugeführt werden kann.

7. Vorrichtung nach Anspruch 6, **dadurch gekennzeichnet, dass** die elektronische Steuereinrichtung (100) so ausgebildet ist, dass sie beim Deaktivieren der Saugpumpe auch die Einrichtung zum Zuführen einer Spülflüssigkeit oder eines sonstigen Fluids deaktiviert.

8. Vorrichtung nach einem oder mehreren der vorstehenden Ansprüche, **dadurch gekennzeichnet, dass** die elektronische Steuereinrichtung (100) so ausgebildet ist, dass sie bei zutreffender oder unzutreffender Erkennung eines Behälter-voll-Zustands die Saugpumpe (90) nicht deaktiviert, sondern den vorgegebenen Unterdruckregelbetrieb fortsetzt.

9. Vorrichtung nach einem oder mehreren der vorstehenden Ansprüche, **dadurch gekennzeichnet, dass** die elektronische Steuereinrichtung (100) so ausgebildet ist, dass sie bei einem Unterdruckanstieg, das heißt bei einer Abnahme des absoluten Drucks in dem Leitungsabschnitt (88) zwischen Behälter (10) und Saugpumpe (90), der insbesondere auf eine zunehmende Befüllung des Behälters schließen lässt, die Saugpumpe (90) nicht deaktiviert, sondern den vorgegebenen Unterdruckregelbetrieb fortsetzt.

10. Verfahren zum Betreiben einer Vorrichtung nach einem oder mehreren der vorstehenden Ansprüche, **dadurch gekennzeichnet, dass** unter Verwendung einer optoelektronisch wirkenden Sensoreinrichtung (120) und der elektronischen Steuereinrichtung (100) festgestellt wird, wenn der Behälter (10) während des laufenden Unterdruckregelbetriebs von dem ersten Gehäuseteil (4) abgenommen wird, und dass die elektronische Steuereinrichtung (100) dann die Saugpumpe (90) deaktiviert.

## Claims

1. Portable device (2) that can be carried on the body of a user for providing a vacuum for medical vacuum treatment of wounds on the body of a person or of an animal, comprising a first housing part (4) with a suction pump (90), which generates a vacuum and is located in the first housing part (4) of the device, a second housing part (8) that forms a container (10) for receiving body fluids, in particular, wound exudate extracted from a wound by suction, wherein the second housing part that forms the container (10) can be detachably mounted to the first housing part (4) of the device and can be evacuated by the suction pump (90) in the mounted state, wherein the housing parts (4, 8) rest against each other via a substantially vertical separating plane (14) and thereby come to rest next to each other in the properly assembled state of the device (2) wherein the two housing parts (4, 8) are altogether designed in the form of a disk, i.e. their width B in the horizontal direction and their height H in the vertical direction are each larger than their depth T in the horizontal direction and perpendicular to the width such that the housing parts (4, 8) disposed next to each other can be positioned on the body in such a fashion that the second housing part (8) faces the body, i.e. comes to rest between the body and the first housing part (4) and the first housing part (4) comes to rest facing away from the body, i.e. substantially forms the visible side of the device 2, and wherein a connection (12) for a suction line (82) leading to the body is provided on the container (10), such that vacuum communication can be established between the suction pump (90), the container (10) and the suction line (82) that leads to the body, a programmable electronic control device (100) which can drive the suction pump (90), thereby at least taking into consideration predetermined and/or predeterminable parameters and pressure values measured by the pressure sensor (94), **characterized in that** a optoelectronically active sensor device (120) is provided by means of which the electronic control device (100) can detect removal of the container (10) from the first housing part (4) during vacuum regulation operation, wherein the sensor device (120) emits a signal, on the basis of which the electronic control device (100) detects removal of the container (10), and the electronic control device (100) is designed in such a fashion that it then deactivates the suction pump (90).

2. Device according to claim 1, **characterized in that** an output or display device (108) is provided for generating a signal which visually or acoustically communicates removal of the container.

3. Device according to any one or more of the preceding claims, **characterized in that** an output or display device is provided which is designed for wireless transmission of a signal to an external receiver, which signal communicates removal of the container to an external receiver.

4. Device according to any one or more of the preceding claims, **characterized in that** the sensor device (120) and the electronic control device (100) are designed in such a fashion that arrangement of the container (10) in its intended position on the first housing part (4) is determined and, starting from a previous deactivation of the suction pump (90), the previously adjusted pressure regulation operation is restarted.

5. Device according to any one or more of the preceding claims, **characterized by** a venting valve (116) that can be controlled by the electronic control device (100) and can be connected to the wound area via a venting line (112) provided in addition to the suction line, such that the wound area can be vented with air from the outside.

6. Device according to one or more of the preceding claims, **characterized by** a device, which can be controlled by the electronic control device (100) for supplying a rinsing liquid or another fluid, which can be connected to the wound area via a rinsing line provided in addition to the suction line such that rinsing liquid or another fluid can be supplied to the wound area.

7. Device according to claim 6, **characterized in that** the electronic control device (100) is designed in such a fashion that, upon deactivation of the suction pump, it also deactivates the device for supplying a rinsing liquid or any other fluid.

8. Device according to any one or more of the preceding claims, **characterized in that** the electronic control device (100) is designed in such a fashion that it does not deactivate the suction pump (90) upon correct or incorrect detection of a full state of the container but continues the predetermined vacuum regulation operation.

9. Device according to any one or more of the preceding claims, **characterized in that** the electronic control device (100) is designed in such a fashion that, in case of a vacuum increase, i.e. a reduction of the absolute pressure in the line section (88) between the container (10) and the suction pump (90) which suggests, in particular, a rising fill level of the container, it does not deactivate the suction pump (90) but continues the predetermined vacuum regulation operation.

10. Method for operating a device according to any one or more of the preceding claims, **characterized in that** removal of the container (10) from the first housing part (4) during ongoing vacuum regulation operation is detected using an optoelectronically active sensor device (120) and the electronic control device (100), in which case the electronic control device (100) deactivates the suction pump (90).

## Revendications

1. Dispositif (2) pouvant être porté sur le corps d'un utilisateur et destiné à fournir une pression négative pour le traitement médical des plaies par pression négative sur un corps humain ou animal, comprenant une première partie de boîtier (4) pourvue d'une pompe aspirante (90) qui produit une pression négative dans la première partie de boîtier (4), une seconde partie de boîtier (8), laquelle forme un contenant (10) servant à recevoir des liquides corporels, en particulier des sécrétions prélevées d'une plaie par aspiration, la seconde partie de boîtier (8) formant le contenant (10) pouvant être fixée amovible sur la première partie de boîtier (4) du dispositif, et, une fois fixée, pouvant être soumise à la pression négative créée par la pompe aspirante (90), les parties de boîtier (4, 8) reposant l'une contre l'autre par l'intermédiaire d'un plan de séparation (14) sensiblement vertical et, une fois le dispositif (2) assemblé conformément aux prescriptions, venant se placer l'une à côté de l'autre, les deux parties de boîtier (4, 8) étant réalisées dans l'ensemble en forme de disque, ce qui signifie que leur largeur (B) dans la direction horizontale et leur hauteur (H) dans la direction verticale sont respectivement supérieures à leur hauteur (T) dans la direction horizontale et perpendiculairement au sens de la largeur, de sorte que les parties de boîtier (4, 8) juxtaposées peuvent être positionnées sur le corps de telle manière que la seconde partie de boîtier (8) vient se placer en direction du corps, par conséquent entre le corps et la première partie de boîtier (4), et la première partie de boîtier (4) vient se placer à l'opposé du corps, par conséquent forme sensiblement la face visible du dispositif (2), et un raccord (12) destiné à une conduite d'aspiration (82) menant au corps étant situé sur le contenant (10), de manière à pouvoir établir une communication de pression négative entre la pompe aspirante (90), le contenant (10) et la conduite d'aspiration (82) menant au corps, un appareil de commande électronique programmable (100), lequel peut commander la pompe aspirante (90) au moins en tenant compte de paramètres prédéfinis et/ou pouvant être prédéfinis, et de valeurs de pression mesurées par le capteur de pression (94), un appareil de détection (120) à action optoélectronique permettant d'établir, par l'intermédiaire de l'appareil de commande électronique (100), si le contenant (10) est retiré de la première partie de boîtier (4) pendant le mode de régulation de pression négative, l'appareil de détection (120) délivrant un signal à l'aide duquel l'appareil de commande électronique (100) détecte le retrait du contenant (10), et en ce que l'appareil de commande électronique (100) est conçu pour alors désactiver la pompe aspirante (90).

2. Dispositif Selon la revendication 1, **caractérisé en ce qu'**un appareil de sortie ou d'affichage (108) permet de produire un signal, lequel fournit une indication visuelle ou acoustique du retrait du contenant.

3. Dispositif selon l'une ou plusieurs des revendications précédentes, **caractérisé en ce qu'**un dispositif de sortie ou d'affichage est conçu pour la transmission sans fil d'un signal à un récepteur externe, lequel indique le retrait du contenant à un récepteur externe.

4. Dispositif selon l'une ou plusieurs des revendications précédentes, **caractérisé en ce que** l'appareil de détection (120) et l'appareil de commande électronique (100) sont conçus pour établir que le contenant (10) est disposé dans sa position conforme aux prescriptions sur la première partie de boîtier (4), et **en ce qu'**à partir d'une désactivation précédemment mise en oeuvre de la pompe aspirante (90), le mode régulation de pression précédemment suspendu est alors repris.

5. Dispositif selon l'une ou plusieurs des revendications précédentes, **caractérisé par** une soupape d'aération (116) pouvant être commandée par l'appareil de commande électronique (100), laquelle soupape peut être reliée à l'espace de plaie au moyen d'une conduite d'aération (112) prévue en plus de la conduite d'aspiration, de sorte que l'espace de plaie peut être aéré par de l'air extérieur.

6. Dispositif selon l'une ou plusieurs des revendications précédentes, **caractérisé par** un dispositif pouvant être commandé par l'appareil de commande électronique (100) et destiné à amener un liquide de lavage ou un autre fluide, lequel dispositif peut être relié à l'espace de plaie par l'intermédiaire d'une conduite de lavage prévue en plus de la conduite d'aspiration, de sorte que le liquide de lavage ou un autre fluide peut être amené dans l'espace de plaie.

7. Dispositif selon la revendication 6, **caractérisé en ce que** l'appareil de commande électronique (100) est conçu, lors de la désactivation de la pompe aspirante, pour désactiver également le dispositif permettant d'amener un liquide de lavage ou un autre fluide.

8. Dispositif selon l'une ou plusieurs des revendications précédentes, **caractérisé en ce que** l'appareil de commande électronique (100) est conçu, lorsqu'un état de contenant plein est détecté de manière correcte ou fausse, pour ne pas désactiver la pompe aspirante (90), mais poursuivre le mode régulation de pression négative prédéfini.

9. Dispositif selon l'une ou plusieurs des revendications précédentes, **caractérisé en ce que** l'appareil de commande électronique (100) est conçu, lors d'une augmentation de la pression négative, c'est-à-dire lors d'une baisse de la pression absolue dans la section de conduite (88) située entre le contenant (10) et la pompe aspirante (90), laquelle peut suggérer en particulier un remplissage croissant du contenant, pour ne pas désactiver la pompe aspirante (90), mais poursuivre le mode de régulation de pression négative prédéfini.

10. Procédé permettant de faire fonctionner un dispositif selon l'une ou plusieurs des revendications précédentes, **caractérisé en ce qu'**on établit au moyen d'un appareil de détection (120) à effet optoélectronique et de l'appareil de commande électronique (100) si le contenant (10) est retiré de la première partie de boîtier (4) pendant le déroulement du mode de régulation de pression négative, et **en ce que** l'appareil de commande électronique (100) désactive alors la pompe aspirante (90).
